# EUROPÄISCHE PATENTANMELDUNG

(11) **EP 0 821 966 A2**
(43) Veröffentlichungstag der Anmeldung: **04.02.1998**
(21) Anmeldenummer: 97112986.1
(22) Anmeldetag: 29.07.1997
(51) Int. Cl.: A61K 33/10

(54) **Mischung enthaltend gekörnt vermahlenen Orangencalcit**

(30) Priorität: 31.07.1996 DE 19630894
(71) Anmelder: Schirrmacher, Jörg, 59229 Ahlen (DE)
(72) Erfinder: Schirrmacher, Jörg, 59229 Ahlen (DE)
(74) Vertreter: Meyers, Hans-Wilhelm, Dr.Dipl.-Chem.

(57) **Zusammenfassung**

Es wird eine Mischung aus gekörntem Orangencalcit beschrieben. Die erfindungsgemäß erhältliche Mischung dient als Badezusatz oder Arzneimittel zur Behandlung von Hauterkrankungen und/oder zur Linderung von mit Hauterkrankungen verbundenen Beschwerden.

## Beschreibung

Die vorliegende Erfindung betrifft eine Mischung enthaltend gekörnt vermahlenen Orangencalcit, einen Badezusatz und ein Arzneimittel enthaltend die Mischung sowie Verwendung einer Mischung enthaltend gekörnt vermahlenen Orangencalcit.

Calcit wird in der Kosmetik eingesetzt. So betrifft die EP-A-0 257 458 ein abrasives Mittel und ein Verfahren zur Herstellung desselben. Dabei wird ein abrasives Mittel umfassend granuliertes Calcit, welches von Gletscherkalkstein extrahiert wurde, verwendet.

Die EP-A-0 571 193 betrifft eine viskose Flüssigkeit, die als Hautreinigungszusammensetzung Verwendung findet. Diese Lösung enthält neben oberflächenaktiven Mitteln abrasive Partikel und Verdickungsmittel. Die Viskosität der Zusammensetzung ist ähnlich derjenigen eines Duschgels und variiert im Bereich von 4.000 bis 8.000 mPas. Die abrasiven Partikel bestehen vorzugsweise aus Calcit mit einem mittleren Durchmesser von 40 bis 400 µm und einer Schüttdichte von 1 bis 4.

JP-A-07291852 beschreibt Badezusätze enthaltend Calcit und gepulverte, getrocknete Meeralgen, die in einem wasserdurchlässigen Beutel in das Badewasser gegeben werden zur Behandlung von Hauterkrankungen.

Die beschriebenen Calcit enthaltenen Mittel gemäß Stand der Technik sind jedoch für eine Hautbehandlung, insbesondere in Badezusätzen oder anderen kosmetischen Zubereitungen zur Linderung oder Therapie von Hauterkrankungen, deswegen nachteilhaft, weil sie aufgrund ihrer Alkalität dazu neigen, selbst hautreizend zu wirken.

Überraschenderweise wurde gefunden, daß eine Mischung enthaltend gekörnt vermahlenen Orangencalcit die Nachteile des bislang verwendeten Calcits nicht aufweist.

Orangencalcit kommt als gelblich-orange auftretendes Mineral in Lagerstätten vor. Fundgebiete des edlen Orangencalcits liegen in Mexiko.

Vorzugsweise besteht die erfindungsgemäße Mischung aus einer Mischung von mindestens zwei Fraktionen mit unterschiedlicher Körnung. Die erste Fraktion enthält dabei eine feinere Korngröße als diejenige der zweiten Fraktion. Typischerweise weist die erste Körnung eine Korngröße von mindestens 0,1 mm auf. Es ist insbesondere bevorzugt, daß die Korngröße der zweiten Fraktion 0,5 bis 3,5 mm beträgt. Der Korngrößenbereich ist nach oben hin begrenzt durch die Tatsache, daß zu große Calcitkörner eine zu starke Abrasion bewirken können, wohingegen eine zu niedrige Korngröße zu einer insgesamt unbrauchbaren Abmischung führt. Es ist insbesondere bevorzugt, eine Korngrößenverteilung der ersten Fraktion von 0,2 bis 1,8 mm und bei der zweiten Fraktion eine Korngröße von 0,5 bis 3,5 mm einzuhalten.

Die relativen Mengenverhältnisse zwischen der ersten und zweiten Fraktion, ausgedrückt in Gew.-%, liegt zwischen 1 : 99 und 1 : 1. Die Größenverhältnisse der einzelnen Fraktionen werden beispielsweise durch Sieben entsprechend gemahlener Fraktionen bestimmt. Die oben beschriebenen Korngrößen sind dabei als Ausschlußgrenzen der jeweils zu verwendenden Siebe zu verstehen. Erfindungsgemäß können jedoch auch die entsprechenden Fraktionen mit ähnlicher, mittleren Korngrößenverteilung eingesetzt werden. Dann ist vorzugsweise darauf zu achten, daß der mittlerer Korngrößendurchmesser den angegebenen Werten entspricht. Es können aber auch Fraktionen mit gleichmäßiger Korngrößenverteilung eingesetzt werden.

Eine bevorzugte Abmischung besteht aus beispielsweise fünf Fraktionen gemahlenen Orangencalcits. Dabei weist die erste Fraktion eine Korngröße von 0,2 bis 0,6 mm, die zweite Fraktion eine Korngröße von 0,4 bis 1,1 mm, die dritte Fraktion eine Korngröße von 0,9 bis 1,8 mm, die vierte Fraktion eine Korngröße von 1,6 bis 2,3 mm und die fünfte Fraktion eine Korngröße von 2,0 bis 3,5 mm auf. Dabei beträgt beispielsweise das Verhältnis die Mengen der ersten Fraktion (Schüttvolumen) 7 bis 15 %, der zweiten Fraktion 13 bis 18 %, der dritten Fraktion 15 bis 22 %, der vierten Fraktion 20 bis 35 % und der fünften Fraktion 30 bis 45 %.

Die erfindungsgemäße Mischung eignet sich überraschenderweise als Zusatz zur Behandlung von Hauterkrankungen oder Linderung von Beschwerden, die mit Hauterkrankungen verbunden sind. Dabei kann die Mischung erfindungsgemäß insbesondere als Badezusatz eingesetzt werden, wobei die erfindungsgemäße Mischung im einfachsten Falle ohne weitere Hilfsstoffe einem entsprechenden Badeansatz (normales Badewasservolumen) zugesetzt wird. Es kann jedoch auch mit hautverträglichen, nicht reizenden oder nicht allergenen Stoffen eine entsprechend zubereitete Bademischung hergestellt werden. Es können insbesondere auch verträgliche Duftstoffe zugefügt werden.

Die erfindungsgemäße Mischung ist auch zur Linderung oder Behandlung von Hauterkrankungen geeignet. Dabei wird die erfindungsgemäße Mischung in einer galenischen Zubereitung, insbesondere für topikale Anwendungen, eingesetzt. Dies kann beispielsweise in Form von Lotionen oder Cremes erfolgen.

Im allgemeinen können den erfindungsgemäßen Zusätzen oder Arzneimitteln geeignete Hilfs- und Trägerstoffe zugefügt werden, die dem Galeniker als solche bekannt sind. Es wird hierbei auf die entsprechende pharmazeutische Literatur ausdrücklich verwiesen. Erfindungsgemäß beansprucht wird auch eine Verwendung der erfindungsgemäßen Mischungen zur Behandlung von Hauterkrankungen und/oder Linderung von Beschwerden, die mit Hauterkrankungen verbunden sind.

Als Hauterkrankungen, die mit den erfindungsgemäßen Mischungen behandelt werden, oder deren Beschwerden gelindert werden können, sind insbesondere Kontaktallergien, Neurodermitis, Psoriasis, allgemeine trockene Haut, allergische Hautreizungen und/oder durch Infektionen hervorgerufenen Hautreizungen zu nennen.

Die Erfindung wird anhand der folgenden Beispiele näher erläutert.

### Beispiel

Fünf Fraktionen von gekörntem Orangencalcit mit folgender Korngrößenverteilung:
Erste Fraktion von 0,2 bis 0,6 mm,
zweite Fraktion von 0,4 bis 1,1 mm,
dritte Fraktion von 0,9 bis 1,8 mm,
vierte Fraktion von 1,6 bis 2,3 mm,
fünfte Fraktion von 2,0 bis 3,5 mm, werden jeweils
mit einem Schüttvolumen, wie nachstehend wiedergegeben:
erste Fraktion 7 bis 15 %,
zweite Fraktion 13 bis 18 %,
dritte Fraktion 15 bis 22 %,
vierte Fraktion 20 bis 35 %,
fünfte Fraktion 30 bis 45 % vermischt und
zu einer für ein Bad vorbereiteten Badewanne in warmes Wasser gegeben. Die zu behandelnde Person begibt sich in das Bad und verweilt dort für ca. 20 bis 30 min. Danach wird die Haut sanft abgespült und getrocknet.

Diese Verfahrensweise führt zur Linderung von Beschwerden, die mit trockener Haut und Juckreiz verbunden sind.

## Patentansprüche

1. Mischung enthaltend gekörnt vermahlenen Orangencalcit.

2. Mischung nach Anspruch 1, dadurch gekennzeichnet, daß die Körnung aus mindestens zwei Fraktionen unterschiedlicher Körnung von Orangencalcit besteht, wobei eine erste Fraktion eine feinere Korngröße mit einer Korngröße von 0,1 mm aufweist und eine zweite Fraktion mit einer größeren Korngröße als derjenigen der ersten Fraktion vorhanden ist.

3. Mischung nach Anspruch 1 und/oder 2, dadurch gekennzeichnet, daß die Menge der ersten Fraktion zur Menge der zweiten Fraktion zueinander im Verhältnis von 1 - 50 Gew.-% liegt.

4. Mischung nach Ansprüche 1 bis 3, dadurch gekennzeichnet, daß die Korngrößen der Fraktionen als mittlere Korngrößen angegeben sind.

5. Mischung nach mindestens einem der Ansprüche 1 bis 4, dadurch gekennzeichnet, daß mindestens eine Fraktion mit gleichmäßiger Korngrößenverteilung vorliegt.

6. Mischung nach mindestens einem der Ansprüche 1 bis 5, dadurch gekennzeichnet, daß die Körnung aus fünf Fraktionen unterschiedlicher Körnung besteht, wobei die erste Fraktion eine Korngröße von 0,2 bis 0,6 mm,
die zweite Fraktion eine Korngröße von 0,4 bis 1,1 mm,
die dritte Fraktion eine Korngröße von 0,9 bis 1,8 mm,
die vierte Fraktion eine Korngröße von 1,6 bis 2,3 mm
und die fünfte Fraktion eine Korngröße von 2,0 bis 3,5 mm aufweist.

7. Mischung nach einem der Ansprüche 1 bis 6, dadurch gekennzeichnet, daß die Menge der ersten Fraktion zur Menge der zweiten, dritten, vierten und fünften Fraktion, ausgedrückt in Schüttvolumen, in einem Bereich von 7 bis 15% liegt,
die zweite Fraktion in einem Bereich von 13 bis 18 %,
die dritte Fraktion in einem Bereich von 15 bis 22 %,
die vierte Fraktion in einem Bereich von 20 bis 35 %
und die fünfte Fraktion in einem Bereich von 30 bis 45 % liegt.

8. Badezusatz enthaltend eine Mischung nach mindestens einem der Ansprüche 1 bis 7, gegebenenfalls neben Hilfs- und Trägerstoffen.

9. Arzneimittel enthaltend eine Mischung nach mindestens einem der Ansprüche 1 bis 7, gegebenenfalls neben Hilfs- und Trägerstoffen.

10. Arzneimittel nach Anspruch 9, dadurch gekennzeichnet, daß es als äußerlich auftragbares Mittel galenisch zubereitet ist.

11. Verwendung einer Mischung nach mindestens einem der Ansprüche 1 bis 7 zur Behandlung von Hauterkrankungen und/oder Linderung von Beschwerden, die mit Hauterkrankung verbunden sind.

12. Verwendung nach Anspruch 11 zur Behandlung von Kontaktallergien, Neurodermitis, Psoriasis, trockener Haut, allergischer Hautreizungen und/oder durch Infektionen hervorgerufener Hautreizungen.
